(19) Europäisches Patentamt
European Patent Office
Office européen des brevets

(11) Veröffentlichungsnummer: **0 135 720**
**B1**

(12) # EUROPÄISCHE PATENTSCHRIFT

(45) Veröffentlichungstag der Patentschrift: **08.03.89**

(51) Int. Cl.⁴: **C 07 K 7/40**

(21) Anmeldenummer: **84109067.3**

(22) Anmeldetag: **01.08.84**

(54) **Verfahren zur Herstellung von Insulin-Derivaten.**

(30) Priorität: **03.08.83 DE 3327928**

(43) Veröffentlichungstag der Anmeldung:
**03.04.85 Patentblatt 85/14**

(45) Bekanntmachung des Hinweises auf die Patenterteilung:
**08.03.89 Patentblatt 89/10**

(84) Benannte Vertragsstaaten:
**AT BE CH DE FR GB IT LI LU NL SE**

(56) Entgegenhaltungen:
**DE-C-952 208**
**FR-A-1 072 410**

(73) Patentinhaber: **HOECHST AKTIENGESELLSCHAFT, Postfach 80 03 20, D-6230 Frankfurt am Main 80 (DE)**

(72) Erfinder: **Grau, Ulrich, Dr., Zeil 17, D-6238 Hofheim am Taunus (DE)**

EP 0 135 720 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Insulin wird in vivo in Form eines Vorläufers, des Präproinsulins, synthetisiert. Die Präsequenz stellt eine Signalregion für Wechselwirkungen mit Membranen dar und wird noch bei oder aber unmittelbar nach der Synthese abgespalten. Das Proinsulin hingegen ist ein Molekül, das in geringen Mengen in Pankreaseextrakten nachweisbar ist. Bei dessen Umwandlung zum Insulin wird durch ein spezifisches Enzymsystem das C-Peptid abgespalten.

Die Schnittstellen für die Prozessierungsenzyme werden gebildet durch die Sequenzen Arg-Arg an den Positionen B31 und B32 (B30 ist der spätere C-Terminus der B-Kette) und Lys-Arg an der Position 62 (bzw. A1) und 63 (bzw. AO), die sich N-terminal zu der späteren A-Kette befinden. Mit Enzymen mit tryptischer und Carboxypeptidase-B-Aktivität läßt sich in vitro Proinsulin glatt in Insulin umwandeln (Kemmler et al., JBC 246, (1971) 6786-91. Die Proteolyse mit Trypsin oder trypsin-ähnlichen Enzymen allein liefert hingeben bevorzugt jene Zwischenprodukte, die noch eines oder beide Arginine in den Positioner 31 und 32 am C-Terminus der B-Kette tragen, neben jenen Insulin-Abbauprodukten, die B-terminal zu Lysin-(B29)-Insulin (=des-Alanin-(B30)-Schweineinsulin, des-Threonin-(B30)-Humaninsulin) oder zu Arginin-(B22)-Insulin (=desoctapeptid-Insulin) gespalten sind (Chance, in Proc. VII Congress of International Diabetes Fed., (1970); D.E. Steiner et al., Fed. Proc. 33, (1974) 2105-15; J. Markussen in Proc. Symp. on Proinsulin, Insulin, C-Peptide, Tokushima (1978)).

Durch gentechnologische Methoden sind heutzutage Präproinsulin aus Prokaryonten, insbesondere E. coli, zugänglich. Besonders interessant sind dabei solche Stämme, die Proinsuline mit menschlicher Insulinsequenz enthalten. Durch Abänderung der Plasmide ist es darüberhinaus möglich, gezielt neue Seqenzabschnitte zu konstruieren, insbesondere Änderungen an den Positionen 31 - 33 und 61 - 63 der Proinsulinsequenz in der Weise, daß enzymatische oder chemische Spaltstellen zur Freisetzung von Insulin bzw. Insulinderivaten erhalten bleiben. Dazu zählen beispielsweise solche Derivate, die an Stelle eines oder beider Arginine an Position 31 und 32 eine andere basische Aminosäure, also Lysin oder Histidin, enthalten, so daß Enzyme mit tryptischer Spezifität zur Spaltung herangezogen werden können. Es können aber auch Sequenzen eingeführt werden, die Substrate anderer bekannter proteolytischer Enzyme sind oder Sequenzen, an denen eine Spaltung mit chemischen Methoden möglich ist. Voraussetzung für die vorliegende Erfindung ist nur, daß bei geeigneter Spaltung des Proinsulinanalogen ein Insulinderivat entsteht, das keine zusätzliche Aminosäure vor dem N-Terminus der A-Kette aufweist und unter den bis zu drei zusätzlichen Aminosäuren am C-Terminus der B-Kette (Positionen 31 - 33) mindestens eine basische Aminosäure trägt.

Solche Insulinderivate die am C-Terminus der B-Kette zusätzlich Positive Ladungen aufweisen, Verfahren zu deren Herstellung, diese enthaltende Mittel und deren Verwendung sind Gegenstand der deutschen Patentanmeldungen DE-A-3 326 472 und DE-A-3 326 473. Ein Verfahren zu ihrer Kristallisation ist Gegenstand der deutschen Patentanmeldung DE-A-3 327 709.

Daß das Insulin in der Nähe seines isoelektrischen Punkten am besten kristallisiert ist z. B. aus DE-C-952 208 bekannt. Die Zugabe von kristallisationsfördernden Phenolen ist in FR-A-1 072 410 beschrieben.

Es wurde nun gefunden, daß diese Insulinderivate besonders glatt aus den entsprechenden Intermediaten, Proinsulinen, Präproinsulinen oder den entsprechenden Analogen herstellbar sind, indem man die proteolytische Verdauung oder die chemische Spaltung in der Nähe des isoelektrischen Punktes des gewünschten Derivats Gegenwart von Phenol oder ähnlichen Aromaten durchführt. Es wird auf diese Weise nicht nur erreicht, daß das Endprodukt aus der Reaktionslösung ausfällt und somit weitgehend dem Reaktionsgleichgewicht entzogen wird; unter den beschriebenen Bedingungen entstehen direkt in dem Medium scharfkantige, prismenförmige Kristalle, die wegen der geringen Oberfläche einer Weiterreaktion viel weniger unterliegen als z. B. amorphe Niederschläge. So ist es beispielsweise erstaunlich, daß das Derivat Insulin-$Arg^{B31}$-OH in kristalliner Form gegen weiteren tryptischen Abbau außerordentlich stabil ist.

Die Erfindung betrifft daher ein Verfahren zur Herstellung von Insulin-Derivaten der Formel I,

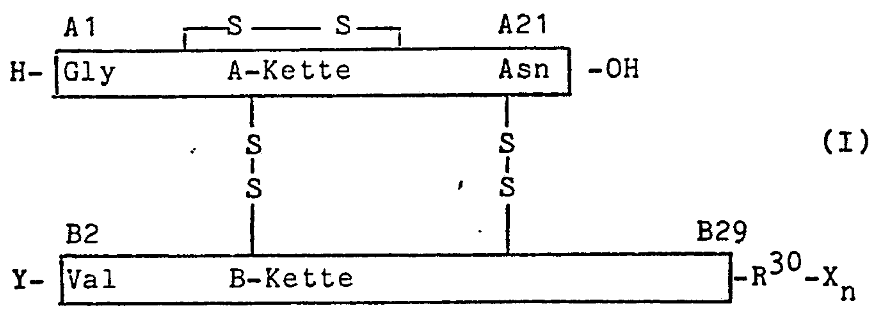

(I)

in welcher

$R^{30}$ für den Rest einer neutralen, genetisch kodierbaren L-Aminosäure steht,

X für gleiche oder verschiedene Reste genetisch kodierbarer L-Aminosäuren steht, darunter mindestens eine basische Aminosäure wobei das C-terminale Glied der Homoserinlactonrest sein kann,

Y = Phe oder H und
n = 1, 2 oder 3 bedeutet,
durch Spaltung von Intermediaten, Proinsulin, Präproinsulin oder deren Analogen, das dadurch gekennzeichnet ist, daß die Spaltreaktion in der Nähe des isoelektrischen Punktes der Insulin-Derivate der Formel I in Gegenwart einer aromatischen Hydroxyverbindung durchgeführt wird.

Bevorzugt ist Y = Phe.

Genetisch kodierbar sind die folgenden L-Aminosäuren: Gly, Ala, Ser, Thr, Val, Leu, Ile, Asp, Asn, Glu, Gln, Cys, Met, Arg, Lys, His, Tyr, Phe, Trp, Pro (neutrale Aminosäuren unterstrichen.

Unter einer neutralen Aminosäure versteht man dann insbesondere Gly, Ala, Ser, Thr, Val, Leu, Ile, Asn, Gln, Met, Phe oder Pro; unter einer basischen Aminosäure versteht man insbesondere Arg, Lys oder His.

Nach dem erfindungsgemäßen Verfahren lassen sich bespielsweise folgende Insulin-Derivate herstellen (ohne daß die Erfindung auf diese beschränkt wäre):

Humaninsulin-$Arg^{B31}$-OH
Schweineinsulin-$Arg^{B31}$-OH
Rinderinsulin-$Arg^{B31}$-OH
Humaninsulin-$Arg^{B31}$-$Arg^{B32}$-OH
Schweineinsulin-$Arg^{B31}$-$Arg^{B32}$-OH
Rinderinsulin-$Arg^{B31}$-$Arg^{B32}$-OH
Humaninsulin-$Lys^{B31}$-OH
Humaninsulin-$Ala^{B31}$-$Lys^{B32}$-OH
Humaninsulin-$Val^{31}$-$Arg^{B32}$-OH
Humaninsulin-$Leu^{B31}$-$Arg^{B32}$-$Arg^{B33}$-OH
Schweineinsulin-$Val^{B31}$-$His^{B32}$-$Arg^{B33}$-OH
Schweineinsulin-$Leu^{B31}$-$Val^{B32}$-$Arg^{B33}$-OH
Humaninsulin-$Arg^{B31}$-$Phe^{B32}$-OH

Humaninsulin-Arg -NH 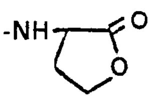

Das erfindungsgemäße Verfahren wird bevorzugt im pH-Bereich von einer pH-Einheit unterhalb des isoelektrischen Punktes bis einer Einheit oberhalb diese Punktes in der Gegenwart eines Phenols oder eines Gemisches mehrerer Phenole durchgeführt.

Zur Einstellung des pH-Wertes können geeignete Puffer (wie Acetate, Citrat oder Phosphat) verwendet werden.

Bevorzugt wird nach dem erfindungsgemäßen Verfahren die Reaktion so geführt, daß die Konzentration des Ausgangsmaterials 0,5 - 30 mg/ml, vorzugsweise 1 - 10 mg/ml beträgt und die Reaktionsgeschwindigkeit geringfügig größer oder gleich ist wie die Bildungsgeschwindigkeit der Kristalle. Auf diese Weise wird erreicht, daß viele Kristallkeime entstehen und damit die Kristallgröße mit Mittel 30 µm nicht übersteigt. Die Reaktionsgeschwindigkeit kann vor allem bei Enzymkatalyse sehr genau in der gewünschten Weise kontrolliert werden.

Mit dem beschriebenen Verfahren ist es also möglich, die Reaktion besser in Richtung auf das gewünschte Produkt zu lenken und damit die Ausbeute deutlich zu verbessern. Das Produkt fällt in einer leicht weiter zu verarbeitender Form an, die von überschüssigen Spaltungsreagens z. B. Proteasen durch einfaches Waschen weitgehend befreit werden kann.

Als Spaltungsreagenz wird beispielsweise eine Protease gewählt, deren Spezifität so ist, daß der gewünschte Rest $X_n$ erhalten bleibt, während die A-Kette nach erfolgter Spaltung einen freien N-Terminus H-Gly aufweist. Werden in der Proinsulinsequenz Methionine an den entsprechenden Stellen eingeführt, so kann die Spaltung mit Bromcyan erfolgen, wobei C-terminal der Homoserinlactonrest erhalten bleibt.

Insulin-Derivate der Formel I und diesen enthaltende pharmazeutische Mittel stellen vollkommen neue Verzögerungsprinzipien dar, die ohne Depot-Hilfsstoffe, wie Zink oder Protaminsulfat, zur Wirkung gebracht werden können. Die Depot-Wirkung geht auf ein inhärentes, proteinchemisch bedingtes physikalisches Prinzip, die Schwerlöslichkeit des Insulin-Derivats am isoelektrischen Punkt, zurück. Seine Wiederauflösung unter physiologischen Bedingungen wird möglicherweise durch Abspaltung der zusätzlichen basischen Gruppen erreicht, die je nach Derivat durch tryptische oder Trypsin-ähnliche, und/oder Carboxypeptidase B- oder Carboxypeptidase B-ähnliche und/oder Esterase-Aktivität zustande kommt.

## Beispiel 1

Tryptische Verdauung von Desnonapeptid-Proinsulin vom Schwein in Abwesenheit und in Gegenwart von Phenol.

350 mg Desnonapeptid-Schweineproinsulin wurden sauer gelöst und zu 215 ml 0,1 M Tris-Puffer pH = 7,5 gegeben, der 200 µg Trypsin (Rind) enthält. Innerhalb weniger Minuten trat eine Trübung auf. Nach beendeter

Reaktion (ca. 1 h) wurde 100 µg Trypsin-Inhibitor zugegeben, zentrifugiert, der weiße Niederschlag in 20 ml 0.05 M Ammoniumacetatpuffer pH = 4,0 mit 0,1 % eines geeigneten Detergens gelöst und am Kationenaustauscher mit einem 0 bis 1 M Kochsalzgradienten gereinigt. Es wurden isoliert: 96 mg (36,6 %) Insulin-$Arg^{B31}$ -$Arg^{B32}$-OH in reiner Form und 72 mg (27,4 %) eines Gemischs aus Insulin-$Arg^{B31}$-$Arg^{B32}$-OH und Insulin-$Arg^{B31}$-OH und mindestens zwei weiteren Verunreinigungen (nach isoelektrischer Fokussierung und Hochdruck-Flüssigkeitschromatographie (HPLC)). Aus dem Überstand der Reaktion wurden 56 mg (ca. 21 %) eines Gemischs mit 14,9 % Ausgangssubstanz, 8,7 % Di- und Monoarginin-Derivat, 49,3 % Des-Alanin(B30)-Insulin und 12,2 % Desoctapeptid-Insulin, neben einigen kleineren, unidentifizierten Peaks (HPLC) isoliert.

In einem Parallelansatz wurden 100 mg Desnonapeptid-Schweineproinsulin sauer gelöst, und zu 50 ml eines 0,1 M Tris-Puffers pH = 7,5 gegeben, der 2,5 mg/ml m-Kresol und 50 µg Trypsin (Rind) enthielt. Nach etwa 15 Minuten bildete sich ein kristalliner Niederschlag aus scharfkantigen Prismen von ca. 5 - 15 µm Größe. Die Reaktion wurde nach 3 h in der beschriebenen Weise abgebrochen. Die Aufarbeitung erfolgte wie oben.

Ausbeuten: 56 mg (74,7 %) Insulin-$Arg^{B31}$-$Arg^{B32}$-OH
11 mg (14,7 %) Gemisch aus Di-Arginin- und Mono-Arginin-Derivat

Im Überstand befindet sich nur noch eine Spur an Insulinähnlichen Substanzen.

**Beispiel 2**

Herstellung von Humaninsulin-$Arg^{B31}$-OH aus Affen-Präproinsulin, exprimiert in E.coli, durch tryptische Verdauung.

10 mg Affen-Präproinsulin werden sauer gelöst und zu 5 ml 0,1 M Phosphatpuffer mit 0,5 M Kochsalz pH = 6,8, der 1,5 mg/ml Phenol und 0,7 mg/ml m-Kresol, sowie 25 µg Trypsin (Rind) enthält, zugegeben. Aus der Reaktionslösung fällt das gewünschte Produkt in scharfkantigen Prismen von ca. 5 - 20 µm Größe aus. Die Kristalle enthalten 92 % an Insulin und Insulin-Derivaten davon nach HPLC 78 % Humaninsulin-$Arg^{B31}$-OH und 14 % Humaninsulin-$Arg^{B31}$-$Arg^{B32}$-OH. Im klaren Überstand der Reaktion wird nur weniger als 5 % an Insulinähnlichem Protein gefunden. Läßt man die Kristallsuspension in Gegenwart von Trypsin noch weitere 2 Tage bei Raumtemperatur stehen, so befinden sich noch immer 87 % der theoretischen Insulin- und Insulin-Derivat-Menge im kristalinen Zustand, davon 79 % Humaninsulin-$Arg^{B31}$-OH und 11 % Humaninsulin-$Arg^{B31}$-$Arg^{B32}$-OH.

**Patentansprüche**

1. Verfahren zur Herstellung von Insulin-Derivaten der Formel I,

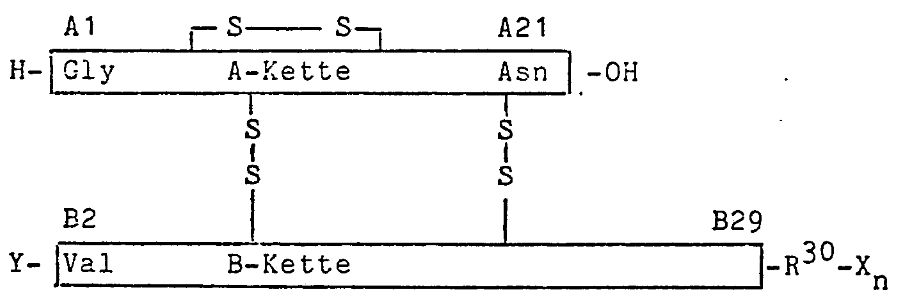

(I)

in welcher

$R^{30}$ für den Rest einer neutralen, genetisch kodierbaren L-Aminosäure steht,
X für gleiche oder verschiedene Reste genetisch kodierbarer L-Aminosäuren steht, darunter mindestens eine basische Aminosäure, wobei das C-terminale Glied Homoserinlacton sein kann,
Y Phe oder H,
n = 1, 2 oder 3 bedeutet,

durch Spaltung von Intermediaten, Proinsulin, Präproinsulin oder deren Analogen, dadurch gekennzeichnet, daß die Spaltreaktion in der Nähe des isoelektrischen Punktes der Insulin-Derivate der Formel I in Gegenwart einer aromatischen Hydroxyverbindung durchgeführt wird.

2. Verfahren gemäß Anspruch 1, dadurch gekennzeichnet, daß $R^{30}$ in der Formel I für Ala, Thr oder Ser steht.

3. Verfahren gemäß Anspruch 1 oder 2, daduerch gekennzeichnet, daß Y in Formel I für Phe steht.

4. Verfahren gemäß einem der Ansprüche 1 bis 3, dadurch gekennzeichnet, daß das Insulin-Derivat der Formel I die Sequenz des Humaninsulin, des Schweineinsulin oder des Rinderinsulin besitzt.

5. Verfahren gemäß einem der Ansprüche 1 bis 4, dadurch gekennzeichnet, daß X in Formel I für einen neutralen und/oder basischen Aminosäurerest steht.

6. Verfahren gemäß einem der Ansprüche 1 bis 5, dadurch gekennzeichnet, daß die Spaltreaktion in Gegenwart von einem Phenol oder eines Gemisches mehrerer Phenole durchgeführt wird.

7. Verfahren gemäß einem der Ansprüche 1 bis 6, dadurch gekennzeichnet, daß die Reaktionsgeschwindigkeit so gewählt wird, daß bevorzugt das Reaktionsprodukt in Form von bis zu 30 µm großen Kristallen anfällt.

8. Verfahren gemäß einem der Ansprüche 1 bis 7, dadurch gekennzeichnet, daß zu Beginn der Reaktion das Intermediat, das Proinsulin oder deren Analoges in einer Konzentration von 0,5 bis 30 mg/ml im Reaktionsmedium vorliegen.

9. Verfahren gemäß einem der Ansprüche 1 bis 8, dadurch gekennzeichnet, daß das Reaktionsmedium ein wäßriges, in geeigneter Weise gepuffertes Medium darstellt.

10. Verfahren gemäß einem der Ansprüche 1 bis 9, dadurch gekennzeichnet, daß das Spaltungsreagenz eine Protease darstellt, deren Spezifität so ist, daß der gewünschte Rest $X_n$ erhalten bleibt, während die A-Kette nach erfolgter Spaltung einen freien N-Terminus aufweist.

11. Verfahren gemäß einem der Ansprüche 1 bis 9, dadurch gekennzeichnet, daß das Spaltungsreagenz Bromcyan darstellt, wodurch am C-Terminus der B-Kette der Homoserinlactonrest erhalten bleibt, während bei der Spaltung an Met-AO der natürliche N-Terminus der A-Kette entsteht.

## Claims

1. A process for the preparation of an insulin derivative of the formula I

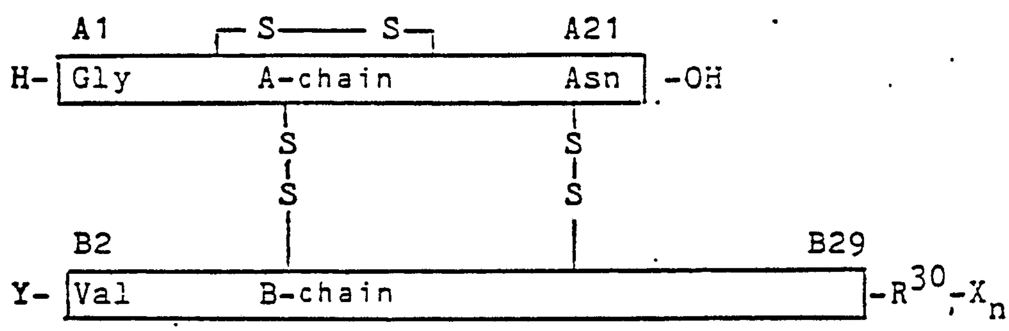

(I)

in which

$R^{30}$ represents the radical of a neutral L-amino acid which can be genetically coded,

X represents identical or different radicals of L-amino acids which can be genetically coded, at least one of which is a basic amino acid, it being possible for the C-terminal member to be homoserine-lactone,

Y = Phe or H and

n = 1, 2 or 3,

by splitting of intermediates, proinsulin, preproinsulin or analogs thereof, which comprises carrying out the splitting reaction close to the isoelectric point of the insulin derivatives of the formula I in the presence of an aromatic hydroxy compound.

2. The process as claimed in claim 1, wherein $R^{30}$ in formula I represents Ala, Thr or Ser.

3. The process as claimed in either of claims 1 or 2, wherein Y in formula I represents Phe.

4. The process as claimed in any one of claims 1 to 3, wherein the insulin derivative of the formula I has the sequence of human insulin, porcine insulin or bovine insulin.

5. The process as claimed in any one of claims 1 to 4, wherein X in formula I represents a neutral and/or basic amino acid radical.

6. The process as claimed in any one of claims 1 to 5, wherein the splitting reaction is carried out in the presence of a phenol or a mixture of several phenols.

7. The process as claimed in any one of claims 1 to 6, wherein the rate of reaction is chosen such that the reaction product is preferentially obtained in the form of crystals up to 30 µm in size.

8. The process as claimed in any one of claims 1 to 7, wherein the intermediate, the proinsulin or the analog thereof is present in the reaction medium in a concentration of 0.5 to 30 mg/ml at the start of the reaction.

9. The process as claimed in any one of claims 1 to 8, wherein the reaction medium is an aqueous medium buffered in a suitable manner.

10. The process as claimed in any one of claims 1 to 9, wherein the splitting reagent is a protease, the specificity of which is such that the desired radical $X_n$ is retained, whilst the A chain has a free N-terminal after splitting has been carried out.

11. The process as claimed in any one of claims 1 to 9, wherein the splitting reagent is cyanogen bromide, the homoserine-lactone radical being retained on the C-terminal of the B chain, while the natural N-terminal of the A chain is formed on splitting at Met-AO.

**Revendications**

1. Procédé pour la préparation de dérivés d'insuline de formule I,

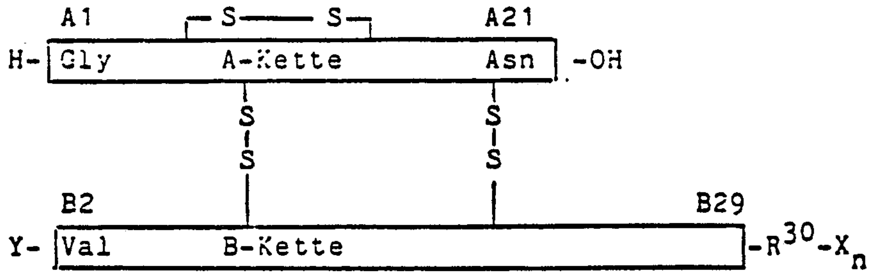

(I)

dans laquelle

$R^{30}$ représente le reste d'un L-aminoacide neutre, génétiquement codable

X représente des restes identiques ou différents de L-aminoacides génétiquement codables, parmi lesquels au moins un aminoacide basique, le chaînon C-terminal pouvant être le reste homosérinelactone,

Y représente Phe ou H, et

n vaut 1, 2 ou 3,

par coupure de produits intermédiaires, proinsuline, préproinsuline ou de leurs analogues, caractérisé en ce que la réaction de coupure est effectuée au voisinage du point isoélectrique du dérivé d'insuline de formule 1, en présence d'un composé aromatique hydroxylé.

2. Procédé selon la revendication 1, caractérisé en ce que $R^{30}$ dans la formule I représente Ala, Thr ou Ser.

3. Procédé selon la revendication 2, caractérisé en ce que Y dans la formule I représente Phe.

4. Procédé selon l'une des revendications 1 à 3, caractérisé en ce que le dérivé d'insuline de formule I possède la séquence de l'insuline humaine, de l'insuline porcine ou de l'insuline bovine.

5. Procédé selon l'une des revendications 1 à 4, caractérisé en ce que X dans la formule I représente un reste aminoacide basique et/ou neutre.

6. Procédé selon l'une des revendications 1 à 5, caractérisé en ce que la réaction de coupure est effectuée en présence d'un phénol ou d'un mélange de plusieurs phénols.

7. Procédé selon l'une des revendications 1 à 6, caractérisé en ce que la vitesse de réaction est choisie de manière que, de préférence, le produit de réaction précipite sous forme de cristaux ayant une taille allant jusqu'à 30 µm.

8. Procédé selon l'une des revendications 1 à 7, caractérisé en ce qu'au début de la réaction, le produit intermédiaire, la proinsuline ou son analogue, sont présents à une concentration de 0,5 à 30 mg/ml dans le milieu réactionnel.

9. Procédé selon l'une des revendications 1 à 8, caractérisé en ce que le milieu réactionnel représente un milieu aqueux, tamponné de façon appropriée.

10. Procédé selon l'une des revendications 1 à 9, caractérisé en ce que le réactif de coupure consiste en une protéase dont la spécificité est telle que le reste $X_n$ désiré est conservé, tandis que la chaîne A présente, une fois effectuée la coupure, une terminaison N libre.

11. Procédé selon l'une des revendications 1 à 9, caractérisé en ce que le réactif de coupure consiste en le bromure de cyanogène, ce par quoi le reste homosérinelactone est conservé à la terminaison C de la chaîne B, tandis que la terminaison N naturelle de la chaine A se forme au niveau de Met-AO lors de la coupure.